# EUROPÄISCHE PATENTANMELDUNG

(11) **EP 3 178 829 A1**
(43) Veröffentlichungstag der Anmeldung: **14.06.2017**
(21) Anmeldenummer: 16201606.7
(22) Anmeldetag: 01.12.2016
(51) Int. Cl.: C07F 15/00, C07B 41/06, C07C 391/02, C07C 45/50, B01J 31/28, C07F 11/00

(54) **ORGANODIARYLSELENOXIDE SOWIE VERFAHREN ZU DEREN HERSTELLUNG**

(30) Priorität: 07.12.2015 EP 15198150
(71) Anmelder: Evonik Degussa GmbH, 45128 Essen (DE)
(72) Erfinder: Dyballa, Katrin Marie, 45657 Recklinghausen (DE); Franke, Robert, 45772 Marl (DE); Weilbeer, Claudia, 06406 Bernburg (DE); Börner, Armin, 18059 Rostock (DE); Selent, Detlef, 18059 Rostock (DE)

(57) **Zusammenfassung**

Neue Organodiarylselenoxide sowie Verfahren zu deren Herstellung als auch deren Verwendung als Ligand in Komplexen.

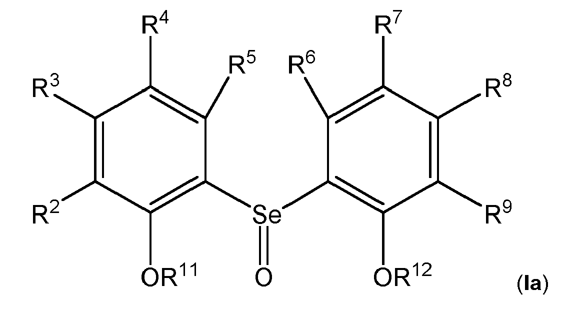

## Beschreibung

Neue Organodiarylselenoxide sowie Verfahren zu deren Herstellung als auch deren Verwendung als Ligand in Komplexen.

Die Herstellung von an der Hydroxylgruppe ungeschützten Selenodiphenolen mit geringen Ausbeuten ist bekannt aus T. K. Paine et al., "Manganese complexes of mixed O, X, O-donor ligands (X = S or Se): synthesis, characterization and catalytic reactivity", Dalton Trans., 2003, 15, 3136-3144. T. K. Paine et al. beschreibt eine Synthese von 2,2'-Selenobis(4,6-di-tert-butylphenol) unter Verwendung von Selendioxid. Die Herstellung von 2,2'-Selenobis(4,6-di-tert-butylphenol) erfolgt hier in einem sauren Medium unter Zusatz von konzentrierter Salzsäure. Das Produkt wird mit einer Ausbeute von nur 25 % erhalten.

Eine weitere mehrstufige Syntheseroute unter Verwendung von Grignard-Reagenz offenbart H. M. Lin et al., "A novel and efficient synthesis of selenides", ARKIVOC, 2012, viii, 146-156. Es wird eine Syntheseroute für Selenobiarylether offenbart in der zunächst Brom an das entsprechende Phenol addiert werden muss, um das Produkt dann mit Magnesium zu einem Grignard-Reagenz umzusetzen. Das Grignard-Reagenz kann dann mit dem zugesetzten Selen reagieren, bevor die eigentliche Kupplung zum Biarylether erfolgt:

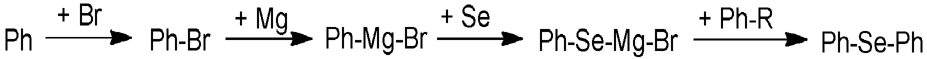

Das Produkt konnte in einer guten Ausbeute erzielt werden, jedoch ist diese Syntheseroute sehr aufwendig, was es für einen großtechnischen Einsatz unattraktiv macht. Hierbei sind eine Vielzahl von Syntheseschritten notwendig, die zum Teil nicht unkritisch in ihrer Durchführung sind, insbesondere wenn man an eine Hochskalierung denkt und Massstäbe verwendet, die in der Industrie üblich sind. Weiterhin fallen bei dieser Syntheseroute große Mengen an Abfallprodukten und Lösungsmittel an, die aufwändig entsorgt werden müssen, unter anderem auf Grund des Einsatzes von Brom.

Eine grosstechnisch wirtschaftliche Syntheseroute zur Herstellung von Selenodiphenolen beschreibt die EP 15168645.8 bzw. US 14/720,063.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxidierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Rhodium-Monophosphit-Komplexe in katalytisch aktiven Zusammensetzungen sind geeignet für die Hydroformylierung von verzweigten Olefinen mit innenständigen Doppelbindungen. Seit den 1970er Jahren ist die Verwendung von so genannten "bulky phosphites" in der Hydroformylierung beschrieben (siehe u.a. van Leeuwen et al., Journal of Catalysis, 2013, 298, 198-205). Diese zeichnen sich durch eine gute Aktivität aus, jedoch ist die n/i-Selektivität für endständig oxidierte Verbindungen gering und verbesserungswürdig.

In diesen Hydroformylierungen werden in der Regel Mono- und Bisphosphite eingesetzt, die oftmals aus Biphenol-Bausteinen aufgebaut sind. Die Entwicklung neuer Liganden ist häufig durch die zur Verfügung stehenden Biphenol-, also Ligandenbausteine limitiert. So stellen 2,2'-Selenobiarylether sowie Diphenylselenoxide und Diphenylselenide eine hochinteressante Verbindungsklasse dar. Die 2,2'-Selenobiarylether werden derzeit nur in bestimmten Komplexen, vor allem manganhaltigen, verwendet, sie besitzen aber ein großes Potential für weitere Anwendungen.

Aufgabe der Erfindung war es, eine weitere gänzlich neue Substanzklasse an Liganden und Ligandenbausteinen herzustellen, um das Feld der verfügbaren Liganden für die jeweiligen spezifischen Komplexe in der Katalyse zu erweitern. Des Weiteren bestand die Aufgabe Liganden für Rhodium-Hydroformylierungskatalysatoren herzustellen. Daher bestand auch die Aufgabe neue Zwischenprodukte als Ligandenbausteine zur Herstellung von Liganden bereitzustellen.

Gelöst wird die Aufgabe durch Organodiarylselenoxide nach Anspruch 1.

Gegenstand der Erfindung ist somit mindestens eine Verbindung eines Organodiarylselenoxids, welches eine allgemeine Struktur **(la)** aufweist 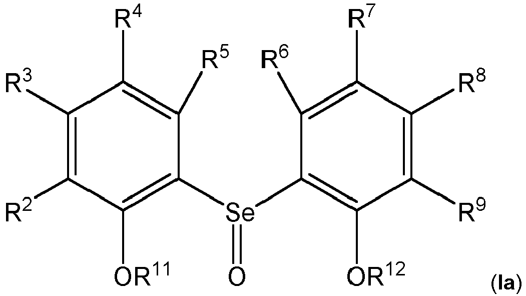
wobei R², R³, R⁴, R⁷, R⁸ und R⁹ in Struktur **(Ia)** jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl,
-CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
   -(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, Fluor, Chlor, Cyano, Formyl, Acyl oder Alkoxycarbonyl,
wobei R⁵ und R⁶ in Struktur **(Ia)** jeweils unabhängig voneinander ausgewählt sind aus: -H, -(C₁-C₁₂)-Alkyl, -O-(C₂-C₁₂)-Alkyl, -(C₆-C₂₀)-Aryl, -O-(C₆-C₂₀)-Aryl, -Halogen, -OC=O-(C₁-C₁₂)-Alkyl, - S-Alkyl, -S-Aryl, -COO-(C₁-C₁₂)-Alkyl,
-CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
   -(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, Fluor, Chlor, Cyano, Formyl, Acyl oder Alkoxycarbonyl,
wobei -R¹¹ und -R¹² jeweils in Struktur **(Ia)** unabhängig voneinander ausgewählt sind aus:
-H, -(C₁-C₁₂)-Alkyl, -(C₁-C₁₂)-Alkyl-O-(C₁-C₁₂)-Alkyl, -(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind,
wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
-(C₆-C₂₀)-Aryl, Fluor, Chlor, Cyano, Formyl, Acyl oder Alkoxycarbonyl.

Alle Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter
-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl. Diese Definition gilt für alle substituierten Alkyl- oder Alkyoxygruppen der vorliegenden Erfindung.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und
-(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltigen Gruppen sind vorzugsweise ausgewählt unter -O-,-S-, -N-, -N(=O)-, -C(=O)- oder -S(=O)-. Beispiele für -(C₃-C₁₂)-Heterocycloalkylgruppen sind Tetrahydrothiophenyl, Tetrahydrofuryl, Tetrahydropyranyl und Dioxanyl.

Halogen umfasst Fluor, Chlor, Brom und Jod, wobei Chlor und Fluor besonders bevorzugt sind.

Eine erfindungsgemässe Alkylgruppe kann jeweils unabhängig voneinander linear, verzweigt oder cyclisch sein.

Ein oder mehrere Substituenten umfassen vorzugsweise 1 bis 10 Substituenten, insbesondere 1 bis 3. Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte
-(C₁-C₈)-Alkyl- und ganz bevorzugt um
-(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl,
2-Methylbutyl-, 3-Methylbutyl-, 1,2-Dimethylpropyl-, 1,1-Dimethylpropyl-,
2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-,
1,3-Dimethylbutyl-, 2,3-Dimethylbutyl-, 3,3-Dimethylbutyl-, 1,1,2-Trimethylpropyl-,
1,2,2-Trimethylpropyl-, 1-Ethylbutyl-, 1-Ethyl-2-methylpropyl-, n-Heptyl-, 2-Heptyl-,
3-Heptyl-, 2-Ethylpentyl-, 1-Propylbutyl-, n-Octyl-, 2-Ethylhexyl-, 2-Propylheptyl-, Nonyl-, Decyl.

In einer Alternative sind R², R³, R⁴, R⁵, R⁶, R⁷, R⁸ und R⁹ jeweils unabhängig voneinander ausgewählt aus: -H, -(C₁-C₁₂)-Alkyl, -O-(C₁-C₁₂)-Alkyl, -(C₆-C₂₀)-Aryl, -O-(C₆-C₂₀)-Aryl, -Halogen.

In einer Alternative sind R², R³, R⁴, R⁵, R⁶, R⁷, R⁸ und R⁹ jeweils unabhängig voneinander ausgewählt aus: -H und -(C₁-C₁₂)-Alkyl.

Nach einer besonders bevorzugten Ausführungsform ist das Organodiarylselenoxid ausgewählt aus einer Verbindung welche eine allgemeine Struktur (**Ib**) aufweist 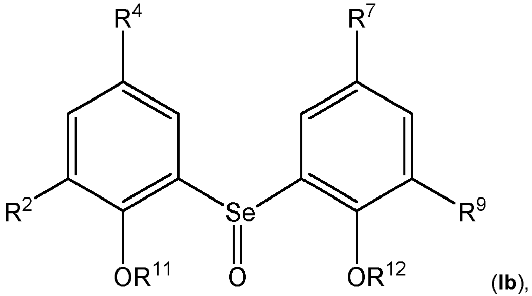 mit R², R⁴, R⁷ und R⁹ in Struktur (**Ib**) gleich -(C₁-C₁₂)-Alkyl,
wobei -R¹¹ und -R¹² jeweils in Struktur (**Ib**) unabhängig voneinander ausgewählt sind aus:
- H, -(C₁-C₁₂)-Alkyl, -(C₁-C₁₂)-Alkyl-O-(C₁-C₁₂)-Alkyl, -(C₆-C₂₀)-Aryl,
- (C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
- (C₆-C₂₀)-Aryl, Fluor, Chlor, Cyano, Formyl, Acyl oder Alkoxycarbonyl.

In einer Ausführungsform sind in dem Organodiarylselenoxid der Strukturen **(Ia)**
R², R³, R⁴, R⁷, R⁸ und R⁹ jeweils unabhängig voneinander ausgewählt aus:
- H, -(C₁-C₁₂)-Alkyl, -O-(C₁-C₁₂)-Alkyl, -(C₆-C₂₀)-Aryl, -O-(C₆-C₂₀)-Aryl, -Halogen,
- OC=O-(C₁-C₁₂)-Alkyl, -S-Alkyl, -S-Aryl, -COO-(C₁-C₁₂)-Alkyl, -CONH-(C₁-C₁₂)-Alkyl,
- CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl-und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
und mindestens ein Rest von R², R³, R⁴, R⁷, R⁸ und R⁹ jeweils unabhängig voneinander ausgewählt ist aus: -(C₆-C₂₀)-Aryl, -O-(C₆-C₂₀)-Aryl, -Halogen,
-OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂,
wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

In einer bevorzugten Ausführungsform sind R¹¹ und R¹² gleich. Ferner sind R¹¹ und R¹² vorzugsweise gleich und ausgewählt aus: -Me, -CH₂OCH₃ (MOM), -CH₂OCH₂C₆H₅ (BOM),-CH₂OCH₂CH₂OCH₃ (MEM), - Benzyl (Bn).

Als Ether-Gruppen nach der Erfindung gelten die Sauerstoffverbrückten Gruppen -OR¹¹ und -OR¹², in denen R¹¹ und R¹² jeweils unabhängig voneinander ausgewählt sind aus:
-(C₁-C₁₂)-Alkyl, -(C₁-C₁₂)-Alkyl-O-(C₁-C₁₂)-Alkyl, -(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl.

Besonders bevorzugt sind R¹¹ und R¹² in den Strukturen **Ia** und **Ib** jeweils unabhängig voneinander ausgewählt aus: -H, Methoxymethyl-, Benzyl-, Methyl-, *tert*.-Butyl.

Ferner sind R¹¹ und R¹² in den Strukturen jeweils unabhängig voneinander ausgewählt aus: -Me, - CH₂OCH₃ (MOM), -CH₂OCH₂C₆H₅ (BOM), -CH₂OCH₂CH₂OCH₃ (MEM), Benzyl-, wobei es weiter bevorzugt ist, wenn R¹¹ und R¹² gleich sind.

Gleichfalls Gegenstand der Erfindung sind Organodiarylselenoxide der Strukturen la mit R², R³, R⁴, R⁵, R⁶, R⁷, R⁸ und R⁹ und **Ib** mit R², R⁴, R⁷und R⁹, die jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl,-CO-(C₆-C₂₀)-Aryl, -COOH, -SO₃H, -CN, -N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, wobei mindestens ein Rest von R², R³, R⁴, R⁵, R⁶, R⁷, R⁸ und R⁹ jeweils unabhängig voneinander ausgewählt ist aus: -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, - SO₃H, -CN, -N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, wobei vorzugsweise R¹¹ und R¹² gleich -H oder R¹¹ und R¹² gleich -OH sind.

Vorzugsweise ist mindestens ein Rest von R², R³, R⁴, R⁵, R⁶, R⁷, R⁸ und R⁹ jeweils unabhängig ausgewählt aus: -(C₆-C₂₀)-Aryl, -O-(C₆-C₂₀)-Aryl, -Halogen, und wobei a) R¹¹ und R¹² gleich -H oder R¹¹ und R¹² gleich -OH sind oder b) R¹¹ und R¹² ungleich sind, wenn R¹¹ oder R¹² gleich -H ist und R¹¹ und R¹² ansonsten der allgemeinen Definition entsprechen.

Ferner ist Gegenstand der Erfindung ein Organodiarylselenoxid
wobei in dem Organodiarylselenoxid
a) der Struktur (**Ia**) oder (**Ib**) R¹¹ gleich R¹² ist und ausgewählt ist aus Methoxymethyl-, Benzyl-, *tert*.-Butyl, oder
b) der Struktur (**Ia**) oder (**Ib**) R¹¹ ausgewählt ist aus Methoxymethyl-, Benzyl-, *tert*.-Butyl, und R¹² gleich - H ist, oder
c) der Struktur (**Ia**) oder (**Ib**) R¹² ausgewählt ist aus Methoxymethyl-, Benzyl-, *tert*.-Butyl und R¹¹ gleich -H ist.

In einer Ausführungsform sind R², R³, R⁴, R⁷, R⁸, R⁹ jeweils unabhängig voneinander ausgewählt aus: -H, -(C₁-C₁₂)-Alkyl, -O-(C₁-C₁₂)-Alkyl, -(C₆-C₂₀)-Aryl und -O-(C₆-C₂₀)-Aryl.

In einer Ausführungsform sind R⁵ und R⁶ jeweils unabhängig voneinander ausgewählt aus: -H, -(C₁-C₁₂)-Alkyl, -O-(C₂-C₁₂)-Alkyl, -(C₆-C₂₀)-Aryl und -O-(C₆-C₂₀)-Aryl.

In einer Ausführungsform sind R², R⁴, R⁷, R⁹ jeweils unabhängig voneinander ausgewählt aus:

-(C₁-C₁₂)-Alkyl, -O-(C₁-C₁₂)-Alkyl, -(C₆-C₂₀)-Aryl und -O-(C₆-C₂₀)-Aryl.

In einer Ausführungsform sind R², R⁴, R⁷, R⁹ jeweils Methyl- oder *tert*-Butyl- und R³, R⁵, R⁶, R⁸ sind jeweils -H.

Entsprechend einer weiteren Ausführungsvariante umfassen die Organodiarylselenoxide der Struktur la auch Organodiphenylselenoxide der allgemeinen Struktur **Ib** 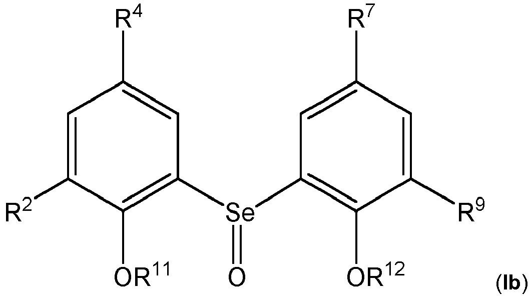 wobei R², R⁴, R⁷ und R⁹ in Struktur **Ib** jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, wobei die genannten Alkyl-und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
-(C₆-C₂₀)-Aryl, und R³, R⁵, R⁶ und R⁸ sind -H, und, wobei in Struktur **Ib** -R¹¹ und -R¹² jeweils -H sind. Die Organodiphenylselenoxide der Struktur **Ib** sind bevorzugt Ligandenbausteine und somit Zwischenprodukte zur Herstellung von Liganden, wie Phosphit-Liganden.

Ferner umfassen in einer Ausführungsvariante auch Organodiphenylselenoxide der allgemeinen Struktur **Ic** 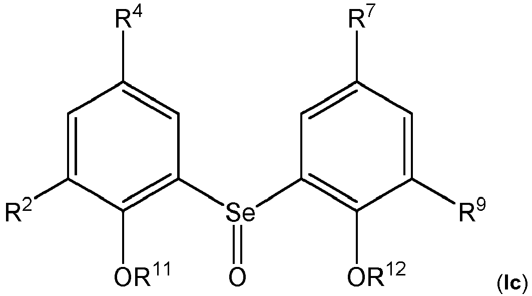 wobei R², R⁴, R⁷ und R⁹ in Struktur Ic jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, und
wobei in Struktur **Ic** -R¹¹ und -R¹² jeweils unabhängig voneinander ausgewählt sind aus:
-(C₁-C₁₂)-Alkyl, -(C₁-C₁₂)-Alkyl-O-(C₁-C₁₂)-Alkyl, -(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl,wobei die genannten Alkyl- und Arylgruppen jeweils unabhängig unsubstituiert sind.

Die Organodiphenylselenoxide der Struktur **Ic** sind bevorzugt Liganden zur Herstellung von Komplexen umfassend mindestens ein Metallatom.

Entsprechend einer besonders bevorzugten Ausführungsvariante sind R², R³, R⁴, R⁵, R⁶, R⁷, R⁸, R⁹ in den Organodiarylselenoxiden, insbesondere den Organodiphenylselenoxiden der Strukturen **Ia**, **Ib**, und/oder **Ic** sowie in den Organodiarylseleniden der Struktur **II** jeweils unabhängig voneinander ausgewählt aus: -H, unsubstituierten -(C₁-C₁₂)-Alkyl und/oder unsubstituierten -O-(C₁-C₁₂)-Alkylgruppen, wobei die Alkylgruppen linear, verzweigt oder cyclisch sein können.

Besonders bevorzugte Ether geschützte Organodiphenylselenoxide der Struktur **Ia**und/oder **Ic**, sind die MOM (Methyloxymethyl) und Bn (Benzyl) geschützten Organodiphenylselenoxide der Struktur **Ic** (**1a** mit R = MOM, und **1b** mit R = Bn).

Weitere bevorzugte Organodiarylselenoxide umfassen Strukturen **Ia**:
(i) mit R², R³, R⁴, R⁵, R⁶, R⁷, R⁸, R⁹ die jeweils unabhängig voneinander ausgewählt sind aus: -H, -(C₁-C₁₂)-Alkyl, -O-(C₁-C₁₂)-Alkyl, -(C₆-C₂₀)-Aryl und -O-(C₆-C₂₀)-Aryl.
(ii) mit R², R³, R⁴, R⁵, R⁶, R⁷, R⁸, R⁹ die jeweils unabhängig voneinander ausgewählt sind aus: - (C₁-C₁₂)-Alkyl, -O-(C₁-C₁₂)-Alkyl, -(C₆-C₂₀)-Aryl und -O-(C₆-C₂₀)-Aryl.
(iii) mit R², R⁴, R⁷, R⁹ die jeweils unabhängig voneinander ausgewählt sind aus: -(C₁-C₁₂)-Alkyl, -O-(C₁-C₁₂)-Alkyl, -(C₆-C₂₀)-Aryl und -O-(C₆-C₂₀)-Aryl, insbesondere der Struktur **Ib** oder **Ic**,
(iv) mit R², R⁴, R⁷, R⁹ die jeweils Methyl- oder tert-Butyl- sind und R³, R⁵, R⁶, R⁸ sind jeweils -H, wobei in den Alternativen (i), (ii), (iii) und (iv) vorzugsweise R¹¹ und R¹² jeweils unabhängig voneinander ausgewählt sind aus: -Me, -CH₂OCH₃ (MOM), -CH₂OCH₂C₆H₅ (BOM), -CH₂OCH₂CH₂OCH₃ (MEM), Benzyl-, wobei es weiter bevorzugt ist, wenn R¹¹ und R¹² gleich sind.

Gleichfalls ist Gegenstand der Erfindung ein Verfahren zur Herstellung eines
Organodiarylselenoxids der allgemeinen
Struktur (**Ia**) umfassend den Verfahrensschritt
(i) Oxidation eines Organodiarylselenids der allgemeinen Struktur (**IIa**) 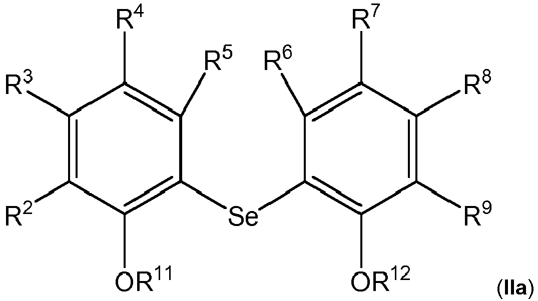
   wobei R², R³, R⁴, R⁷, R⁸ und R⁹ in Struktur **(Ia)** jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl,
   -CONH-(C₁-C₁₂)-Alkyl, -CO-(C₁-C₁₂)-Alkyl, -CO-(C₆-C₂₀)-Aryl, -COOH, -SO₃H, -CN,
   -N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
      -(C₆-C₂₀)-Arylgruppen mindestens einen Substituenten aufweisen und der mindestens eine Substituent jeweils unabhängig ausgewählt ist aus -(C₃-C₁₂)-Cycloalkyl,
   -(C₃-C₁₂)-Heterocycloalkyl, -(C₆-C₂₀)-Aryl, Fluor, Chlor, Cyano, Formyl, Acyl oder Alkoxycarbonyl,
   wobei R⁵ und R⁶ in Struktur **(Ia)** jeweils unabhängig voneinander ausgewählt sind aus: -H, -(C₁-C₁₂)-Alkyl, -O-(C₂-C₁₂)-Alkyl, -(C₆-C₂₀)-Aryl, -O-(C₆-C₂₀)-Aryl, -Halogen, -OC=O-(C₁-C₁₂)-Alkyl, - S-Alkyl, -S-Aryl, -COO-(C₁-C₁₂)-Alkyl,
   -CONH-(C₁-C₁₂)-Alkyl, -CO-(C₁-C₁₂)-Alkyl, -CO-(C₆-C₂₀)-Aryl, -COOH, -SO₃H, -CN,
   -N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
      -(C₆-C₂₀)-Arylgruppen mindestens einen Substituenten aufweisen und der mindestens eine Substituent jeweils unabhängig ausgewählt ist aus -(C₃-C₁₂)-Cycloalkyl,
   -(C₃-C₁₂)-Heterocycloalkyl, -(C₆-C₂₀)-Aryl, Fluor, Chlor, Cyano, Formyl, Acyl oder Alkoxycarbonyl,
   wobei -R¹¹ und -R¹² jeweils in Struktur **(Ia)** unabhängig voneinander ausgewählt sind aus:
   -H, -(C₁-C₁₂)-Alkyl, -(C₁-C₁₂)-Alkyl-O-(C₁-C₁₂)-Alkyl, -(C₆-C₂₀)-Aryl,
   -(C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind,
   wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
   -(C₆-C₂₀)-Aryl, Fluor, Chlor, Cyano, Formyl, Acyl oder Alkoxycarbonyl,
(ii) mindestens eine Verbindung eines Organodiarylselenoxids der allgemeinen Struktur **(Ia)** erhalten wird, 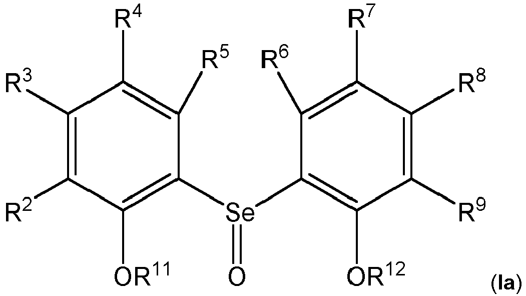
   wobei R², R³, R⁴, R⁷, R⁸ und R⁹ in Struktur (**Ia**) jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, - OC=O-(C₁-C₁₂)-Alkyl, -S-Alkyl, -S-Aryl, -COO-(C₁-C₁₂)-Alkyl,
   -CONH-(C₁-C₁₂)-Alkyl, -CO-(C₁-C₁₂)-Alkyl, -CO-(C₆-C₂₀)-Aryl, -COOH, -SO₃H, -CN,
   -N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
      -(C₆-C₂₀)-Arylgruppen mindestens einen Substituenten aufweisen und der mindestens eine Substituent jeweils unabhängig ausgewählt ist aus -(C₃-C₁₂)-Cycloalkyl,
   -(C₃-C₁₂)-Heterocycloalkyl, -(C₆-C₂₀)-Aryl, Fluor, Chlor, Cyano, Formyl, Acyl oder Alkoxycarbonyl,
   wobei R⁵ und R⁶ in Struktur **(Ia)** jeweils unabhängig voneinander ausgewählt sind aus: -H, -(C₁-C₁₂)-Alkyl, -O-(C₂-C₁₂)-Alkyl, -(C₆-C₂₀)-Aryl, -O-(C₆-C₂₀)-Aryl, -Halogen, -OC=O-(C₁-C₁₂)-Alkyl,-S-Alkyl, -S-Aryl, -COO-(C₁-C₁₂)-Alkyl,
   -CONH-(C₁-C₁₂)-Alkyl, -CO-(C₁-C₁₂)-Alkyl, -CO-(C₆-C₂₀)-Aryl, -COOH, -SO₃H, -CN,
   -N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
      -(C₆-C₂₀)-Arylgruppen mindestens einen Substituenten aufweisen und der mindestens eine Substituent jeweils unabhängig ausgewählt ist aus -(C₃-C₁₂)-Cycloalkyl,
   -(C₃-C₁₂)-Heterocycloalkyl, -(C₆-C₂₀)-Aryl, Fluor, Chlor, Cyano, Formyl, Acyl oder Alkoxycarbonyl,
   wobei -R¹¹ und -R¹² jeweils in Struktur **(Ia)** unabhängig voneinander ausgewählt sind aus:
   -H, -(C₁-C₁₂)-Alkyl, -(C₁-C₁₂)-Alkyl-O-(C₁-C₁₂)-Alkyl, -(C₆-C₂₀)-Aryl,
   -(C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
   -(C₆-C₂₀)-Aryl, Fluor, Chlor, Cyano, Formyl, Acyl oder Alkoxycarbonyl.

Zur (i) Oxidation des Organodiarylselenids der allgemeinen Struktur II kann vorzugsweise N-Chlorsuccinimid (CNS), Bromsuccinimid (BNS), Wasserstoffperoxid, *tert*-Butylhypochlorit (tBuOCl), Natriumhypochlorit (NaOCl) und/oder *meta*-Chlorbenzoesäure (mCPBA) verwendet werden. Besonders bevorzugt wird N-Chlorsuccinimid (CNS), Bromsuccinimid (BNS) und insbesondere N-Chlorsuccinimid (CNS) zur Oxidation des Organodiarylselenids der Struktur **II** verwendet.

Entsprechend einer besonders bevorzugten Verfahrensvariante sind R¹¹ und R¹² jeweils unabhängig voneinander ausgewählt sind aus: -(C₁-C₁₂)-Alkyl,
- (C₁-C₁₂)-Alkyl-O-(C₁-C₁₂)-Alkyl, -(C₆-C₂₀)-Aryl, -(C₁₋C₁₂₎-Alkyl-O-(C₆-C₂₀₎-Aryl,
- (C₆-C₂₀)-Aryl-O-(C₆-C₂₀)-Aryl oder R¹¹ oder R¹² ist -H und der jeweils andere Rest von R¹¹ und R¹² ist ausgewählt aus: -(C₁-C₁₂)-Alkyl, -(C₁-C₁₂)-Alkyl-O-(C₁-C₁₂)-Alkyl,
- (C₆-C₂₀)-Aryl, -(C₁-C₁₂)-Alkyl-0-(C₆-C₂o)-Aryl, -(C₆-C₂₀)-Aryl-O-(C₆-C₂₀)-Aryl.

Ebenso ist Gegenstand der Erfindung ein Verfahren in dem ein Diarylselenid der Struktur **IIb** oder **IIc** oxidiert wird 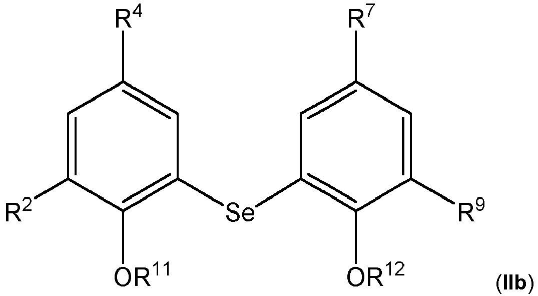
wobei R², R⁴, R⁷ und R⁹ in Struktur **IIb** jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, wobei die genannten Alkyl-und Arylgruppen jeweils unabhängig unsubstituiert oder substituiert sind, wobei substituierte -(C₁-C₁₂)-Alkylgruppen und substituierte
-(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, und R³, R⁵, R⁶ und R⁸ sind -H und
wobei in Struktur **IIb** -R¹¹ und -R¹² jeweils -H sind, und 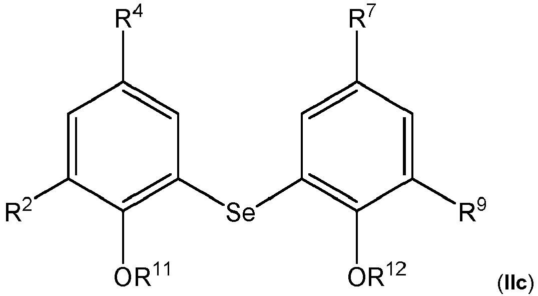
wobei R², R⁴, R⁷ und R⁹ in Struktur **IIc** jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl,
-Halogen, und R³, R⁵, R⁶ und R⁸ sind -H und, wobei in Struktur **IIc** -R¹¹ und -R¹² jeweils unabhängig voneinander ausgewählt sind aus: -(C₁-C₁₂)-Alkyl,
-(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl,
-(C₆-C₂₀)-Aryl-O-(C₆-C₂₀)-Aryl, wobei die genannten Alkyl- und Arylgruppen jeweils unabhängig unsubstituiert sind, wie vorstehend definiert.

Ein weiterer Gegenstand der Erfindung ist ein Komplex umfassend
- mindestens ein Organodiarylselenoxid wie zuvor beschrieben und
- mindestens ein Metallatom ausgewählt aus Rh, Ru, Co, Ir.

Nach einer weiteren Ausführungsform ist Gegenstand der Erfindung ein Komplex umfassend mindestens eine Verbindung der allgemeinen Struktur **Ic** der und mindestens ein Metallatom ausgewählt aus Rh, Ru, Co, Ir, insbesondere Rh, Ir, Ru, bevorzugt Rh. Dabei umfassend die Komplexe vorzugsweise die Struktur **Ic** mit R¹¹ gleich -OR¹¹ und R¹² gleich -OR¹², wobei R¹¹ und R¹² nicht -H sind und R¹¹ und R¹² ansonsten den vorgenannten Definitionen entsprechen, vorzugsweise mit R¹¹ gleich R¹².

Ein weiterer Gegenstand der Erfindung ist die Verwendung mindestens einer Verbindung wie zuvor beschrieben
a) als Ligand in einem Komplex umfassend mindestens ein Metallatom oder
b) zur Katalyse einer Hydroformylierungsreaktion.

Ferner ist Gegenstand der Erfindung die Verwendung eines Organodiarylselenoxids der Struktur **Ic** oder von Gemischen umfassend mindestens zwei der genannten Strukturen, in denen R¹¹ und R¹² nicht -H sind und, wobei R¹¹ und R¹² ansonsten den vorgenannten Definitionen entsprechen, vorzugsweise mit R¹¹ gleich R¹² als Ligand in einem Komplex umfassend mindestens ein Metallatom, vorzugsweise ausgewählt aus Rh, Ru, Co, Ir, insbesondere Rh, Ir, Ru, bevorzugt Rh.

Nach einer weiteren Alternative ist Gegenstand der Erfindung die Verwendung mindestens einer Verbindung der allgemeinen Struktur **Ic** oder von Gemischen umfassend mindestens zwei der genannten Strukturen zur Katalyse einer Hydroformylierungsreaktion, besonders bevorzugt ist die Verwendung einer Verbindung der Struktur **Ic**, in denen R¹¹ und R¹² nicht -H sind und R¹¹ und R¹² ansonsten den vorgenannten Definitionen entsprechen, vorzugsweise mit R¹¹ gleich R¹².

Ferner ist Gegenstand der Erfindung ein Verfahren umfassend die Verfahrensschritte
(i) Vorlegen mindestens eines Olefins,
(ii) Zugabe eines Komplexes umfassend mindestens ein Organodiarylselenoxid der allgemeinen Struktur **Ic** und mindestens ein Metallatom ausgewählt aus Rh, Ru, Co, Ir, insbesondere Rh, Ir, Ru, bevorzugt Rh, und oder ein Organodiarylselenoxid der allgemeinen Struktur **Ic**, und einer Substanz, welche ein Metallatom ausgewählt aus Rh, Ru, Co, Ir aufweist, insbesondere Rh, Ir, Ru, bevorzugt Rh
(iii) Zuführen von H₂ und CO,
(iv) Erwärmen des Reaktionsgemisches,
wobei das Olefin zu einem Aldehyd umgesetzt wird. Die Verfahrensschritte (i), (ii), (iii) und (iv) können alternativ in einer beliebigen Reihenfolge durchgeführt werden.

Dabei kann vorzugsweise mit den erfindungsgemäßen Verbindungen der Strukturen **Ic** in einer Hydroformylierung gemäß vorstehender Verwendung oder vorstehendem Verfahren eine Ausbeute von größer gleich 80 %, insbesondere größer 85%, bevorzugt grösser 90% und/oder eine n-Regioselektivität von größer 15 %, insbesondere grösser 20%, erzielt werden.

Nachfolgend wird die Erfindung näher an Beispielen erläutert, ohne die Erfindung auf die Ausführungsbeispiele zu beschränken.

### Allgemeine Methoden

### Lösungsmittel und Reagenzien

Alle Reaktionen mit feuchtigkeits- und/oder sauerstoffempfindlichen Substanzen wurden in ausgeheizten Apparaturen unter Argonatmosphäre durchgeführt. Lösungsmittel zur Extraktion und Säulenchromatographie wurden in folgenden Reinheiten verwendet: Dichlormethan (99.9%, Fa. *Walter,* Art.-Nr. BIE 073107033) Ethylacetat (99.5%, Fa. *Walter,* Art.-Nr. BIE 003917025) und *n-*Hexan (95%, Fa. *Walter (Baker),* Art.-Nr. 8669),
n-Heptan (95%, Fa. *Walter (Baker),* Art.-Nr. 8662). Andere Lösungsmittel für Extraktion und Säulenchromatographie waren von technischer Qualität und wurden, sofern nicht anders vermerkt, ohne weitere Reinigung eingesetzt. Trockene Lösungsmittel (abs.) wurden mit einem Pure Solv MD-7 System gereinigt und unter Argonatmosphäre aufbewahrt. Benzylbromid wurde vor dem Gebrauch frisch destilliert (17 mbar/82°C). Deuterierte Lösungsmittel wurden von den angegebenen Trockenmitteln destilliert: Dichlormethan-d₂ (Phosphorpentoxid), Toluol-d₈ (1. KOH; 2. Natrium). Für die Synthesen wurden Chemikalien der Firmen *Sigma Aldrich, Alfa Aesar, Acros Organics, Avantor Performance Materials B.V., Merck KGaA* und *ABCR GmbH* & *Co. KG* verwendet. Diese wurden, sofern nicht anders vermerkt, ohne weitere Reinigung eingesetzt.

### Chromatographische Methoden

Die Säulenchromatographie: Säulenchromatographische Trennungen wurden bei erhöhtem Druck (Flash-Chromatographie) an Silicagel 60 230-400 mesh der Firma *Merck KGaA* (Korngröße: 0.040-0.063 mm) durchgeführt. Die verwendeten Eluentengemische sowie die Volumenverhältnisse v/v sind in den nachfolgenden Vorschriften angegeben. Für die verwendeten Eluenten gelten folgende Abkürzungen: DCM (Dichlormethan), EE (Ethylacetat), H (n-Hexan), sowie Tol (Toluol).

Filtration: Filtrationen zur Abtrennung von entstandenen Feststoffen wurden mit einer G4-Fritte (Porenweite: 10-16 µm) durchgeführt.

### Analytik

IR-Spektroskopie: Die IR-Spektren wurden mit dem FT-IR-Spektrometer *Nicolet 6700* der Firma *Thermo Electron* aufgenommen. Die Substanzen wurden mittels ATR-Verfahren vermessen.
¹H-NMR-Spektroskopie: Die ¹H-NMR-Spektren wurden mit dem Modell *AV300* (300 MHz) sowie mit dem Modell *Fourier 300* (300 MHz) der Firma *Bruker* aufgenommen. Die chemischen Verschiebungen sind in Einheiten der δ-Skala angegeben. Die Restprotonensignale der Lösungsmittel (Dichlormethan-d₂: δ = 5.32 ppm, Toluol-d₈:
   δ = 7.09; 7.00; 6.98; 2.09 ppm) dienten dabei als Standard.
¹³ C-NMR-Spektroskopie: Die ¹³C-NMR-Spektren wurden mit den Modellen *AV 300* (75 MHz) und *Fourier 300* (75 MHz) der Firma *Bruker* aufgenommen. Als interner Standard diente das Signal des Lösungsmittels (Dichlormethan-d₂: δ = 54.0 ppm, Toluol-d₈:
   δ = 137.9; 129.2; 128.3; 125.5; 20.4 ppm) wobei die chemischen Verschiebungen den ¹H-breitbandentkoppelten Spektren entnommen wurden.
⁷⁷Se-NMR-Spektroskopie: Die ⁷⁷Se-NMR-Spektren wurden mit dem *AV 300* (57 MHz) der Firma *Bruker* aufgenommen. Die Spektren wurden ¹H-breitbandentkoppelt vermessen. Die chemischen Verschiebungen sind in ppm angegeben.
Massenspektrometrie: EI-Massespektren wurden am Gerät *Finnigan MAT 95-XP* der Firma *Thermo Electron* sowie ESI-TOF-Massespektren mit dem Modell *6210 Time-of Flight LCIMS* der Firma *Agilent* aufgenommen.

### Autoklaven-Versuche der Rhodium-katalysierten Hydroformylierung

Die Hydroformylierung wurde in einem mit Druckkonstanthaltung, Gasflussmessung, Begasungsrührer und Druckpipette ausgestattetem 200 mL-Autoklaven der Fa. *Premex Reactor* AG, Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat 1-Octen oder *n*-Octene (*EVONIK Industries AG,* Octenisomerengemisch aus 1-Octen: 3,3 %; *cis*+*trans-*2*-*Octen: 48.5%; *cis*+*trans-*3-Octen: 29.2%; *cis*+*trans-*Octen-4: 16.4%; gerüstisomere Octene: 2.6%) wurde mehrere Stunden über Natrium am Rückfluss erhitzt und unter Argon destilliert.

Für die Versuche wurden im Autoklaven unter Argonatmosphäre Lösungen der Katalysatorvorstufe und des Liganden gemischt. Als Katalysatorvorstufe kam [(acac)Rh(COD)] (*Umicore,* acac=Acetylacetonat-Anion; COD=1,5-Cyclooctadien) zum Einsatz. Für Versuche mit einer Konzentration von 100 ppm-m Rhodium wurden 10 mL einer 4.31 mM Lösung in den Autoklaven gegeben. Anschließend wurde die einem Verhältnis L/Rh = 5:1 (bzw. 1:1) entsprechende Masse des Liganden in 10 mL Toluol gelöst und zugemischt. Durch Zugabe von weiterem Toluol wurde das Anfangsvolumen der Katalysatorlösung auf 41.0 ml eingestellt. In eine druckfeste Pipette wurde eingefüllt: 1-Octen bzw. n-Octene (10.70 g). Der Autoklav wurde bei einem Gesamtgasdruck (Synthesegas: *Linde;* H₂ (99.999%): CO (99.997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48.5 bar für einen Enddruck von 50 bar bzw. b) 19.5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. *Bronkhorst,* NL) über 4h geführt. Der Autoklav wurde nach Ablauf der Reaktionszeit auf Zimmertemperatur abgekühlt, unter Rühren entspannt und mit Argon gespült. Jeweils 1.0 mL der Reaktionsmischungen wurden unmittelbar nach Abschalten des Rührers entnommen, mit 5.0 mL Pentan verdünnt und gaschromatographisch analysiert: HP 5890 Series II plus, PONA, 50 m x 0.2 mm x 0.5 µm.

**Abkürzungen:** Bn = Benzyl; ber. = berechnet; gef. = gefunden; MOM = Methylmethoxy; NCS = *N-*Chlorsuccimid; RT = Raumtemperatur

### Allgemeine Arbeitsvorschrift zur Synthese der Vorstufen:

### Allgemeine Arbeitsvorschrift (AAV1) zur Darstellung von Organodiarylselenide II als Selenodiphenolen.

Die entsprechenden Phenole (1 Äquivalent) wurden zu einer Mischung aus Selendioxid (0.6 Äquivalente) in Pyridin gegeben und für 2-18 Stunden bei 55-85 °C gerührt. Anschließend wurden die Reaktionsmischungen mit Essigsäureethylester verdünnt, filtriert und die organische Phasen mit Salzsäure (10%) und Wasser gewaschen. Nach Abtrennung der organischen Phase wurde diese über Magnesiumsulfat getrocknet und das Lösungsmittel unter vermindertem Druck abdestilliert. Das Rohprodukt von **II** wurde jeweils säulenchromatographisch gereinigt.

### Bis(3,5-dimethyl-2-hydroxyphenyl)selen, IIb (1)

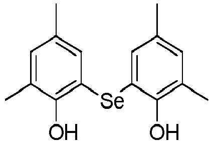

In einem 250 mL Rundkolben wurden 49.9 g Selendioxid (413 mmol) in 100 mL Pyridin mit Hilfe eines Ölbades auf 55 °C erwärmt. Anschließend wurden 25 mL
2,4-Dimethylphenol (206 mmol) hinzugegeben und die Temperatur für siebeneinhalb Stunden gehalten. Nach Beendigung der Reaktion wurde das Gemisch mit 400 mL Ethylacetat verdünnt und filtriert. Die organische Phase wurde mit Wasser gewaschen und über Magnesiumsulfat getrocknet. Das Pyridin wurde destillativ entfernt und der Rückstand erneut in Ethylacetat gelöst und mit 10%iger Salzsäure und Wasser gewaschen um Reste von Pyridin zu entfernen. Die organische Phase wurde mit Magnesiumsulfat getrocknet und unter vermindertem Druck vom Lösungsmittel befreit. Das so erhaltene Rohprodukt wurde in 400 mL Cyclohexan unter Rückfluss erhitzt. Nach Abkühlung auf Raumtemperatur kristallisierte das Produkt. Nach einem Tag wurde das Produkt abfiltriert, das Filtrat auf die Hälfte eingeengt und bei 4 °C erneut zur Kristallisation gebracht. Es wurden 18.56 g, 58 mmol (56%) feine, leicht gelbe Platten des Produktes erhalten mₚ= 120.1 °C (Rekristallisation aus Cyclohexan).
¹H NMR (400 MHz, CDCl₃) δ= 7.11-7.12 (m, 2H), 6.90-6.92 (m, 2H), 5.95 (br, 2H, OH), 2.23 (s, 6H), 2.19 (s, 6H); ¹³C NMR (100 MHz, CDCl₃) δ= 152.04, 133.35, 133.30, 130.67, 124.42, 115.31, 20.45, 16.69; ⁷⁷ Se NMR (76 MHz, CDCl₃) δ= 164.91; HRMS für C₁₆H₁₈O₂⁸⁰Se (ESI+) [M+Na⁺]: berechnet: 345,0370, gefunden: 445.0363;
Elementaranalyse für C₁₆H₁₈O₂Se: berechnet: C: 59.82%, H: 5.65%, gefunden:
   C: 59.69%, H: 5.76%.

### Di-(3-tert-butyl-2-hydroxy-5-methylphenyl)selen, IIb (2)

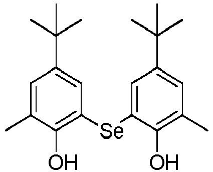

Wie in AAV1 beschrieben wurden 0.80 g 4-*tert*-Butyl-2-methylphenol (4.9 mmol, 1.0 eq) zu einer Lösung von 0.33 g Selendioxid (2.9 mmol, 0.6 eq.) in 6.7 mL Pyridin gegeben und bei 55 °C für 56 Stunden gerührt. Die Reaktionsmischung wurde mit 50 mL Ethylacetat verdünnt, filtriert und dreimal mit je 50 mL Salzsäure (10%) und einmal mit
50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel Cyclohexan/ Ethylacetat 99:1 Ausbeute: 36%, 0.35 g, 0.9 mmol.
mₚ= 98.5 °C; ¹H NMR (400 MHz, CDCl₃) δ= 7.30 (d, ⁴*J*=2.4 Hz, 2H), 7.11 (d, ⁴*J*=2.4 Hz, 2H), 5.92 (s, 2H, OH), 2.26 (d, 6H), 1.23 (s, 18H); ¹³C NMR (100 MHz, CDCl₃) δ= 151.78, 144.03, 129.83, 129.51, 123.89, 114.95, 34.18, 31.56, 16.99; HRMS für C₂₂H₃₀O₂⁸⁰Se (ESI+) [M+Na⁺]:berechnet: 429,1309, gefunden: 429.1250.

### Bis(3,5-di-tert-butyl-2-hydroxyphenyl)selen, IIb (3)

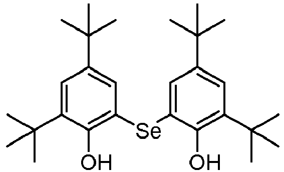

Wie in AAV1 beschrieben wurden 0.80 g 2,4-Di-*tert*-butylphenol (3.8 mmol, 1.0 eq.) zu einer Lösung von 0.25 g Selendioxid (2.3 mmol, 0.6 eq.) in 5.4 mL Pyridin gegeben und bei 55 °C für 4 Tage gerührt. Die Reaktionsmischung wurde mit 50 mL Ethylacetat verdünnt, filtriert und dreimal mit je 50 mL Salzsäure (10%) und einmal mit 50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel Cyclohexan/ Ethylacetat 99:1 Das gewünschte Produkt wurde bei 4 °C aus n-Heptan kristallisiert. Ausbeute: 25%, 0.24 g, 0.5 mmol.
mₚ= 141.1 °C (Rekristallisation aus Heptan); ¹H NMR (400 MHz, CDCl₃) δ= 7.31 (d, ⁴*J*=2.4 Hz, 2H), 7.29 (d, ⁴*J*=2.4 Hz, 2H), 6.29 (s, 2H, OH), 1.42 (s, 18H), 1.24 (s, 18H);
¹³C NMR (100 MHz, CDCl₃) δ= 151.7, 143.5, 135.8, 129.8, 125.6, 117.2, 35.4, 34.4, 31.6, 29.7; HRMS für C₂₈H₄₂O₂⁸⁰Se (ESI+) [M+Na⁺]: berechnet: 513.2248, gefunden: 513.2152.

### Di(3-tert-butyl-5-ethyl-2-hydroxyphenyl)selen, IIb (4)

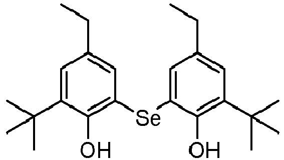

Wie in AAV1 beschrieben wurden 2.00 g 2-*tert*-Butyl-4-ethylphenol (15.8 mmol, 1.0 eq.) zu einer Lösung von 1.06 g Selendioxid (9.5 mmol, 0.6 eq.) in 18 mL Pyridin gegeben und bei 60 °C für 4 Tage gerührt. Die Reaktionsmischung wurde mit 50 mL Ethylacetat verdünnt, filtriert und dreimal mit je 50 mL Salzsäure (10%) und einmal mit 50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel: Cyclohexan/ Ethylacetat 99:1 Ausbeute: 27%, 0.659 g, 1.5 mmol.
mₚ= 68.2 °C; ¹H NMR (400 MHz, CDCl₃) δ= 7.18 (d, ⁴J= 2.1 Hz, 2H), 7.07 (d, ⁴J= 2.1 Hz, 2H), 6.32 (s, 2H, OH), 2.51 (q, ³J= 7.6 Hz, 4H), 1.40 (s, 18H), 1.16 (t, ³J= 7.6 Hz, 6H).
¹³C NMR (100 MHz, CDCl₃) δ= 152.00, 136.42, 136.32, 131.98, 128.22, 117.19, 35.09, 29.53, 28.12, 15.66; HRMS für C₂₄H₂₄O₂⁸⁰Se (ESI+) [M+Na⁺]: berechnet: 457.1622, gefunden: 457.1632; Elementaranalyse für C₂₄H₂₄O₂Se: berechnet: C: 66.50%, H: 7.38%, gefunden: C: 66.26%, H: 7.54%.

### Bis(3,5-di(1,1-dimethylpropyl)-2-hydroxyphenyl)selen, IIb (5)

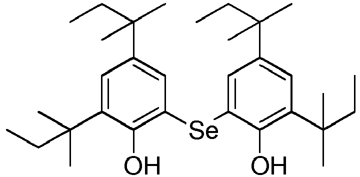

Wie in AAV1 beschrieben wurden 2.00 g 2,4-Di-(1,1-dimethylpropyl)phenol (13.6 mmol, 1.0 eq.) zu einer Lösung von 0.91 g Selendioxid (8.2 mmol, 0.6 eq.) in 19 mL Pyridin gegeben und bei 60 °C für 4 Tage gerührt. Die Reaktionsmischung wurde mit 50 mL Ethylacetat verdünnt, filtriert und dreimal mit je 50 mL Salzsäure (10%) und einmal mit
50 mL Kochsalzlösung gewaschen. Nach dem Trocknen über Magnesiumsulfat wurde das Lösungsmittel unter vermindertem Druck entfernt und der erhaltene Rückstand säulenchromatographisch gereinigt (Laufmittel: Cyclohexan/ Ethylacetat mit einem Gradienten von 100:0 bis 95:5). Ausbeute: 25%, 0,586 g, 1.1 mmol.
mₚ=119.7 °C; ¹H NMR (400 MHz, CDCl₃) δ= 7.18 (d, ⁴*J=* 2.3 Hz, 2H), 7.11 (d, ⁴*J*= 2.3 Hz, 2H), 6.15 (s, 2H), 1.82 (q, ³*J*= 7.5 Hz, 4 H), 1.50 (q, ³*J*= 7.4 Hz, 4 H), 1.34 (s, 12H), 1.16 (s, 12H), 0.58 (q, ³*J*= 7.5 Hz, 12 H); ¹³C NMR (100 MHz, CDCl₃) δ= 151.34, 142.32, 133.78, 130.15, 127.34, 116.99, 38.85, 37.45, 36.94, 33.11, 28.45, 27.72, 9.46, 9.01; HRMS for C₂₂H₃₀O₂⁸⁰Se (ESI+) [M+Na⁺]: berechnet: 569.2874, gefunden: 569.2800.

### Synthese der Hydroxyl-geschützten Selenodiphenole IIc (1a, 1b)

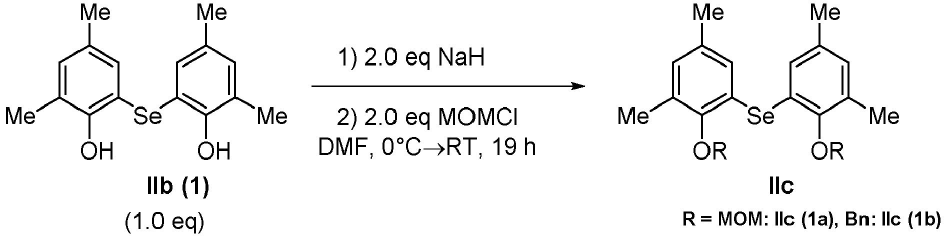

**Beispiel 1:** In einem ausgeheizten, unter Argon-Atmosphäre befindlichen 25 mL Schlenkgefäß wurden 2.0 eq Natriumhydrid (60%ig in Paraffinöl) in 3.0 mL abs. DMF suspendiert und auf 0°C gekühlt. Anschließend wurden 1.0 eq Selenodiphenol **IIb,** gelöst in 2.0 mL abs. DMF, tropfenweise addiert. Die resultierende gelbliche Lösung wurde zehn Minuten bei 0°C und eine Stunde bei RT gerührt. Anschließend wurden erneut bei 0°C, 2.0 eq des Halogenides addiert und zehn Minuten bei 0°C gerührt, wobei eine Trübung der Reaktionslösung beobachtet wurde. Nach weiteren 17 Stunden bei RT wurde unter Eiskühlung Wasser (3.0 mL/1.0 mmol) addiert und die entstehenden Phasen getrennt. Die wässrige Phase wurde mit Ethylacetat (3x je 5.0 mL/1.0 mmol) extrahiert. Die vereinigten organischen Phasen wurden mit Wasser (2x je 10 mL/1.0 mmol) und einer gesättigten NaCl-Lösung (2x je 10 mL/1.0 mmol) gewaschen und über Magnesiumsulfat getrocknet. Das Trockenmittel wurde abfiltriert und das Lösungsmittel unter vermindertem Druck entfernt. Das erhaltene gelbliche Öl wurde in Acetontitril (5.0 mL/1.0 mmol) aufgenommen und mit n-Heptan (2.5 mL/1.0 mmol) versetzt. Die Phasen wurden getrennt und das Lösungsmittel unter vermindertem Druck entfernt. Das Rohprodukt wurde drei Stunden im Vakuum bei 50°C getrocknet.

### Beispiel 2: Synthese von Bis(2-(methoxymethoxy)-3,5-dimethylphenyl)selan IIc (1a)

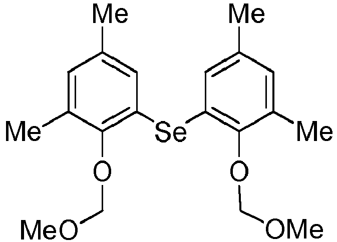

Gemäß Beispiel 1 wurden 81.0 mg (2.03 mmol, 2.0 eq, 60%ig in Paraffinöl) Natriumhydrid und 548 mg (1.70 mmol, 1.0 eq) Selenodiphenol **IIb** mit 153 µL (301 mg, 3.74 mmol, 2.0 eq) Chlordimethylether umgesetzt. Nach extraktiver Aufarbeitung wurden 657 mg (1.60 mmol, 94%) der Titelverbindung **IIc(1a)** als hellgelbes Öl erhalten.
IR (ATR): *v̂* (cm⁻¹) = 2922; 2824; 2772; 1739; 1598; 1568; 1471; 1432; 1395; 1270; 1226; 1194; 1154; 1126; 1069; 951; 924; 849; 814; 796; 757; 727; 581; 540; 511; 477; 440;
¹H-NMR (300 MHz, Dichlormethan-d₂): δ (ppm) = 6.85 (dp, *J* = 2.2 Hz, *J* = 0.7 Hz, 2H,
Ar-CH); 6.75 (dp, *J* = 2.2 Hz, *J* = 0.7 Hz, 2H, Ar-CH); 4.92 (s, 4H, -OCH₂CH₃); 3.50 (s, 6H, -OCH₃); 2.22 (t, *J =* 0.7 Hz, 6H, -CH₃); 2.09 (t, *J* = 0.7 Hz, 6H, -CH₃); ¹³C-NMR (75 MHz, Dichlormethan-d₂): δ (ppm)= 153.4; 135.2; 132.5; 132.1; 131.9; 125.0; 100.0; 57.85; 20.69; 17.25; ⁷⁷Se-NMR (57 MHz, Dichlormethan-d₂): δ (ppm) = 309.0; HR-MS (ESI-TOF): ber. für C₂₀H₂₆O₄SeNa ([M+Na]⁺): 433.08896, gef.: 433.08876;
C₂₀H₂₆O₄Se (410.10 g/mol).

### Beispiel 3: Synthese von Bis(2-(benzyloxy)-3,5-dimethylphenyl)selan IIc (1b)

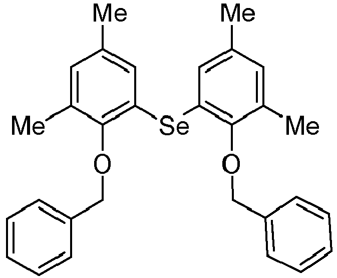

Gemäß Beispiel 1 wurden 82.4 mg (2.06 mmol, 2.0 eq, 60%ig in Paraffinöl) Natriumhydrid und 331 mg (1.03 mmol, 1.0 eq) Selenodiphenol **IIb** mit 244 µL (352 mg, 2.06 mmol, 2.0 eq) Benzylbromid umgesetzt. Nach extraktiver Aufarbeitung wurden 407 mg (0.810 mmol, 79%) der Titelverbindung **IIc(1b)** als hellgelbes Öl erhalten.
IR (ATR): *^{v̂}* (cm⁻¹) = 3088; 3063; 3029; 2917; 2859; 2730; 1598; 1566; 1497; 1465; 1453; 1370; 1308; 1270; 1209; 1127; 1078; 978; 912; 848; 815; 776; 749; 725; 694; 601; 569; 513; 492; 466; ¹H-NMR (300 MHz, Dichlormethan-d₂): δ (ppm) = 7.42-7.30 (m, 4H,
Ar-CH); 7.30-7.09 (m, 6H, Ar-CH); 6.90-6.68 (m, 4H, Ar-CH); 4.78 (s, 4H, -OCH₂Ph); 2.19 (s, 6H,-CH₃); 2.10 (t, *J =* 0.7 Hz, 6H, -CH₃); ¹³C-NMR (75 MHz, Dichlormethan-d₂):
   δ (ppm)= 154.5; 138.0; 135.2; 132.6; 132.0; 131.9; 128.7; 128.5; 128.3; 125.1; 74.77; 20.86; 16.77; ⁷⁷Se-NMR (57 MHz, Dichlormethan-d₂): δ (ppm) = 299.2; ⁷⁷Se-NMR (57 MHz, Toluol-d₈): δ (ppm) = 302.6; MS (ESI-TOF): m/z = 525.130 ([M+Na]⁺); 541.124 ([M+K]⁺); HR-MS (ESI-TOF): ber. für C₃₀H₃₀O₂SeNa ([M+Na]⁺): 525.13053, gef.: 525.12986; C₃₀H₃₀O₂Se (502.14 g/mol).

Sowohl Bis(3,5-dimethyl-2-hydroxyphenyl)selen, Di-(3-*tert*-butyl-2-hydroxy-5-methyl-phenyl)selen; Bis(3,5-di-*tert*-butyl-2-hydroxyphenyl)selen; Di(3-*tert*-butyl-5-ethyl-2-hydroxyphenyl)selen; Bis(3,5-di(1,1-dimethylpropyl)-2-hydroxyphenyl)selen;
Bis(3-*tert*-butyl-5-methyl-2-hydroxyphenyl)selen, Bis(3,3',5,5'-Tetra-*tert*-butyl-2-hydroxyphenyl)selen können analog den Beispielen 1 bis 3 zu den entsprechenden Bis(2-(methoxymethoxy)- oder Bis(2-(benzyloxy)- substituierten Selenen der allgemeinen Struktur II, umgesetzt werden.

### Monoschützung des Selenodiphenols

### Beispiel 4: Synthese von 2-((2-(Benzyloxy)-3,5-dimethylphenyl)selanyl)-4,6-dimethylphenol IIc:

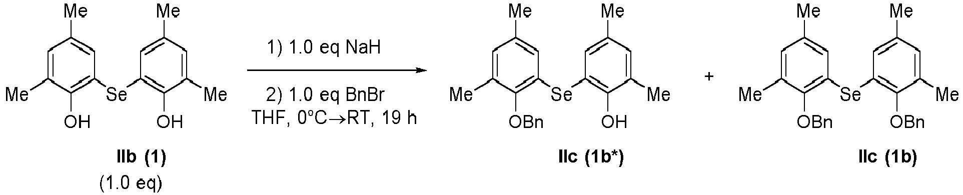

In einem ausgeheizten, unter Argon-Atmosphäre befindlichen 25 mL Schlenkgefäß wurden 40.2 mg (1.01 mmol, 1.0 eq, 60%ig in Paraffinöl) Natriumhydrid in 3.0 mL abs. THF suspendiert und auf 0°C gekühlt. Anschließend wurden 324 mg (1.01 mmol, 1.0 eq) Selenodiphenol **IIb,** gelöst in 2.0 mL abs. THF, tropfenweise addiert. Die gelbliche Lösung wurde 15 Minuten bei 0°C und zwei Stunden bei RT gerührt. Anschließend wurden bei 0°C, 119 µL (172 mg, 1.01 mmol, 1.0 eq) Benzylbromid addiert und 30 Minuten bei 0°C gerührt. Nach weiteren 16 Stunden bei RT wurde das Lösungsmittel unter vermindertem Druck entfernt. Es wurden 391 mg des Reaktionsgemisches aus
2-((2-(Benzyloxy)-3,5-dimethylphenyl)selanyl)-4,6-dimethylphenol **IIc** (**1b***) (314 mg, 0.762 mmol, 76%) und Bis(2-(benzyloxy)-3,5-dimethylphenyl)selan **IIc** (**1b**) (76.6 mg, 0.152 mmol, 15%) in einem Verhältnis von 4.96:1 (bestimmt aus Roh-¹H-NMR-Spektrum) erhalten. Mittels säulenchromatographischer Aufreinigung
(100% H → 100:1 → 50:1 → 20:1 → 10:1 H/DCM) konnten 51.0 mg der Verbindung **IIc** (**1b***) rein isoliert werden, die nachfolgend charakterisiert werden konnte. 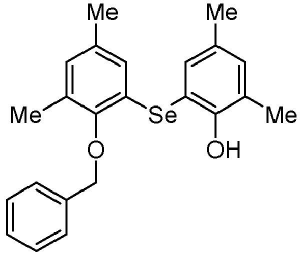
IR (ATR): *v̂* (cm⁻¹) = 3409; 3030; 3011; 2919; 2854; 2730; 1567; 1497; 1467; 1372; 1328; 1284; 1268; 1251; 1231; 1208; 1123; 1078; 1010; 976; 912; 858; 814; 763; 749; 725; 695; 602; 570; 516; 491; 461; ¹H-NMR (300 MHz, Toluol-d₈): δ (ppm) = 7.62-7.43 (m, 2H,
Ar-CH); 7.36-7.29 (m, 1 H, Ar-CH); 7.28-7.08 (m, 2H, Ar-CH); 6.86-6.66 (m, 3H, Ar-CH); 6.58 (d, *J =* 2.1 Hz, 1 H, Ar-CH); 4.82 (s, 2H, -OCH₂Ph); 2.27 (s, 3H, -CH₃); 2.12 (d, *J =* 2.4 Hz, 3H, -CH₃); 2.07 (s, 3H, -CH₃); 1.86 (s, 3H, -CH₃); ¹³C-NMR (75 MHz, Toluol-d₈):
   δ (ppm)= 154.2; 153.2; 137.7; 136.0; 135.1; 134.6; 131.3; 131.2; 129.7; 129.0; 128.6; 128.3; 128.2; 128.1; 74.94; 30.30; 20.53; 20.18; 16.93; 16.31; ⁷⁷Se-NMR (57 MHz,
   Toluol-d₈): δ (ppm) = 207.3; HR-MS (ESI-TOF): ber. für C₂₃H₂₅O₂Se ([M+H]⁺): 413.10155, gef.: 413.10109; ber. für C₂₃H₂₄O₂SeNa ([M+Na]⁺): 435.0835, gef.: 435.08378; C₂₃H₂₄O₂Se (412.09 g/mol).

Die Oxidation von Diphenylselenid **IV** unter den beschriebenen Bedingungen ist literaturbekannt, die übrigen Oxidationen wurden in analoger Weise durchgeführt.

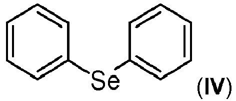

### Synthese der Organodiarylselenoxid-Verbindungen

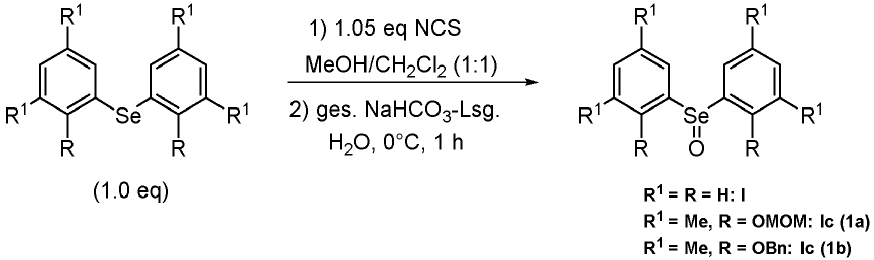

**AAV-2:** In einem 50 mL Zweihalskolben wurden 1.0 eq der Organodiphenylselenid **II** Verbindung in einer 1:1-Mischung aus Dichlormethan/Methanol (13.4 mL/1.0 mmol) gelöst und auf 0°C gekühlt. Anschließend wurden 1.05 eq *N*-Chlorsuccinimid addiert und 30 Minuten bei 0°C gerührt, wobei eine leichte Gelbfärbung der Lösung beobachtet wurde. Anschließend wurde eine gesättigte NaHCO₃-Lösung (10 mL/1.0 mmol) addiert, 15 Minuten gerührt, Wasser (15 mL/1.0 mmol) zugegeben und nochmals für 15 Minuten bei 0°C gerührt. Die Phasen wurden getrennt und die organische Phase mit Wasser (3x je 25 mL/1.0 mmol) gewaschen. Die wässrige Phase wurde mit Dichlormethan (3x je 25 mL/1.0 mmol) extrahiert und über Magnesiumsulfat getrocknet. Das Trockenmittel wurde abfiltriert, das Lösungsmittel unter vermindertem Druck entfernt und das Rohprodukt drei Stunden im Vakuum bei 50°C getrocknet.

### a) Synthese von Seleninyldibenzen III (Verpleichsbeispiel)

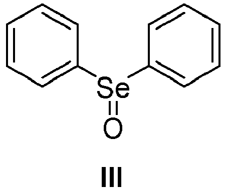

Gemäß **AAV-2** wurden 175 µL (234 mg, 1.00 mmol, 1.0 eq) Diphenylselenid mit 140 mg (1.05 mmol, 1.05 eq) *N*-Chlorsuccinimid umgesetzt. Nach extraktiver Aufarbeitung wurden 235 mg (0.940 mmol, 94%) der Titelverbindung **III** als farbloser Feststoff erhalten.
**IR** (ATR): *v̂* (cm-¹) = 3044; 3008; 2989; 2941; 1570; 1470; 1437; 1300; 1156; 1069; 1056; 1047; 1017; 993; 915; 850; 820; 733; 686; 611; 481; 442,¹**H-NMR** (300 MHz, Toluol-d₈):
   δ (ppm) = 7.67-7.51 (m, 4H, Ar-CH); 7.16-6.87 (m, 6H, Ar-CH); ¹³**C-NMR** (75 MHz, Toluol-d₈): δ (ppm) = 145.1; 130.5; 129.3; 126.0; ⁷⁷**Se-NMR** (57 MHz, Toluol-d₈): δ (ppm) = 851.0; **HR-MS (ESI-TOF):** ber. für C₁₂H₁₁OSe ([M+H]⁺): 250.99700, gef.: 250.99691;
   ber. für C₁₂H₁₀OSeNa ([M+Na]⁺): 272.97894, gef.: 272.97888; **C₁₂H₁₀OSe** (249.99 g/mol).

Die analytischen Daten stammen mit den Literaturdaten überein.

### b) 2-(Benzyloxy)-1-((2-(methoxymethoxy)-3,5-dimethylphenyl)seleninyl)-3,5-dimethylbenzen Ic (1a)

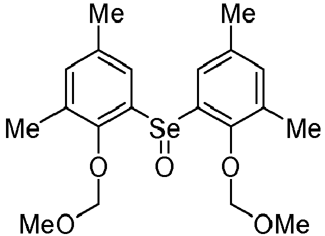

### Gemäß AAV-2 wurden 407 mg (0.991 mmol, 1.0 eq)

Bis(2-(methoxymethoxy)-3,5-dimethylphenyl)selan **IIc (1a)** mit 139 mg (1.04 mmol, 1.05 eq) *N*-Chlorsuccinimid umgesetzt. Nach extraktiver Aufarbeitung wurden 397 mg (0.932 mmol, 94%) der Titelverbindung **Ic (1a)** als farbloser Feststoff erhalten.
¹H-NMR (300 MHz, Dichlormethan-d₂): δ (ppm) = 7.27-7.17 (m, 2H, Ar-CH); 7.12 (td, *J* = 1.4, 0.7 Hz, 2H, Ar-CH); 5.02 (d, *J* = 1.1 Hz, 4H, -OCH₂OCH₃); 3.59 (s, 6H, -OCH₃); 2.29 (d, *J* = 0.7 Hz, 6H, 5-CH₃); 2.28 (t, *J* = 0.6 Hz, 6H, 3-CH₃); ¹³C-NMR (75 MHz, Dichlormethan-d₂): δ (ppm) = 152.3; 137.1; 135.7; 131.6; 125.6; 100.7; 58.14; 0.94; 16.84; ⁷⁷Se-NMR (57 MHz, Toluol-d8): δ (ppm) = 831.0 ppm; C₂₀H₂₆O₅Se (426.09 g/mol).

### c) Synthese von 6,6'-Seleninylbis1-(benzyloxy)-2,4-dimethylbenzen Ic (1b)

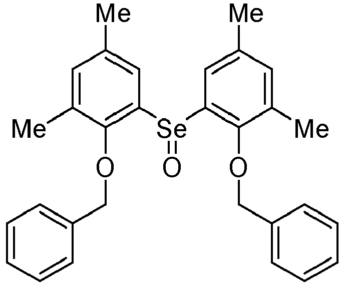

### Gemäß AAV-2 wurden 185 mg (0.369 mmol, 1.0 eq)

Bis(2-(benzyloxy)-3,5-dimethylphenyl)selan **IIc (1b)** mit 51.7 mg (1.05 mmol, 1.05 eq) *N*-Chlorsuccinimid umgesetzt. Nach extraktiver Aufarbeitung wurden 143 mg (0.276 mmol, 75%) der Titelverbindung **Ic (1b)** als farbloser Feststoff erhalten.
IR (ATR): *v̂*(cm⁻¹) = 3376; 3089; 3062; 3031; 3004; 2953; 2920; 2853; 2734; 1588; 1467; 1374; 1364; 1270; 1233; 1219; 1207; 1196; 1116; 1080; 1041; 916; 863; 842; 824; 777; 748; 725; 696; 600; 567; 524; 514; 495; 464; ¹H-NMR (300 MHz, Toluol-d8): δ (ppm) = 7.46 (d, *J* = 2.2 Hz, 1 H, Ar-CH); 7.12-7.03 (m, 4H, Ar-CH); 6.98-6.87 (m, 4H, Ar-CH); 6.83 (p, *J* = 1.1 Hz, 1 H, Ar-CH); 6.81-6.77 (m, 2H, Ar-CH); 6.49 (d, *J* = 2.0 Hz, 2H, Ar-CH); 4.57 (d, *J* = 11.2 Hz, 2H, -CHHPh); 4.39 (d, *J* = 11.3 Hz, 2H-CHHPh); 1.79 (s, 6H, 5-CH₃); 1.75 (s, 6H, 3-CH₃); ¹³C-NMR (75 MHz, Toluol-d₈): δ (ppm) = 154.0; 138.0; 135.5; 135.0; 131.2; 128.4; 128.1; 127.9; 126.6; 125.6; 76.05; 20.58; 16.00; ⁷⁷Se-NMR (57 MHz,
Toluol-d₈): δ (ppm) = 834.2 ppm; HR-MS (ESI-TOF): ber. für C₃₀H₃₁O₃Se ([M+H]⁺): 519.14351, gef.: 519.14372; ber. für C₃₀H₃₀O₃SeNa ([M+Na]⁺): 541.12545, gef.: 541.12524; C₃₀H₃₀O₃Se (518.14 g/mol).

### Katalyse - Hydroformylierung

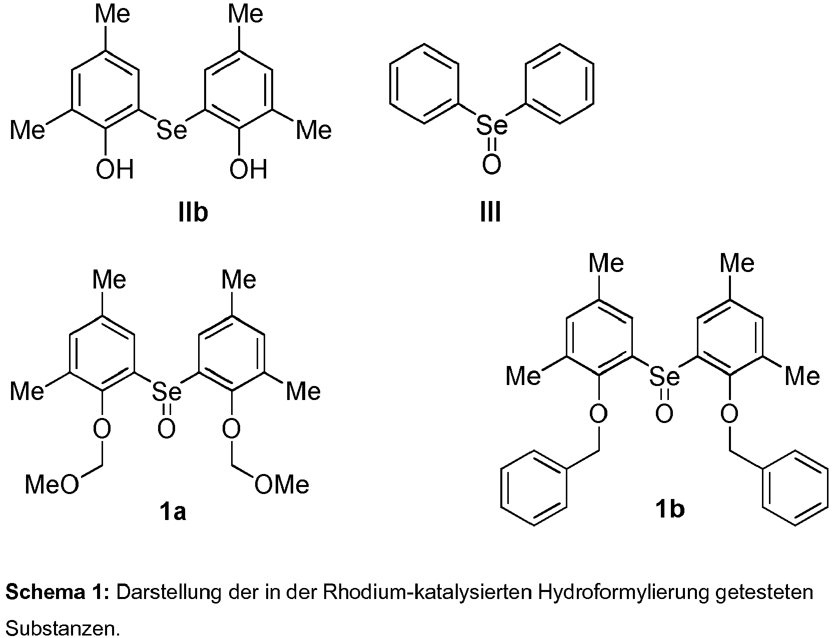

**Tabelle 1: Darstellung der Katalyse-Experimente unter der Verwendung von nicht erfindungsgemäßen Organoselenverbindungen.**

| **Eintrag** | **Ligand** | **Olefin /LM** | **Verhältnis Rh/Ligand/Olefin** | **p [bar]** | **T [°C]** | **t [h]** | **A [%]** | **S [%]** |
|---|---|---|---|---|---|---|---|---|
| 1 | **IIb** | n-Octen / Toluol | 1:1:2197 (100 ppm Rh) | 50 | 120 | 4 | 9.5 | 33.2 |
| 2 | **III** | n-Octen / Toluol | 1:1:2205 (40 ppm Rh) | 50 | 120 | 4 | 89.5 | 28.1 |
| 3* | **1a** | n-Octen / Toluol | 1:1:2203 (100 ppm Rh) | 50 | 120 | 4 | 96,1 | 28,1 |
| 4* | **1b** | n-Octen / Toluol | 1:1:2215 (100 ppm Rh) | 50 | 120 | 4 | 96,7 | 28,4 |

| | | | | | | | | |
|---|---|---|---|---|---|---|---|---|
| ***Erläuterungen zu Tabelle 1**: p = Druck, T = Temperatur, t = Zeit, A = Ausbeute; S = n-Regioselektivität,* * *= erfindungsgemäß* | | | | | | | | |

Die Rhodium-katalysierte Hydroformylierung mit einem ungeschützten Selenodiphenolen **IIb** (Eintrag 1) führt zu einer geringen Ausbeute von 9.5% (Erhalt von 90.5% Restolefin) und einer n-Regioselektivität von 33.2%.

Der Katalyse-Versuch 2) verdeutlicht den erfolgreichen Einsatz der unsubstituierten Diphenylselenoxid-Verbindung in der Rhodium-katalysierten Hydroformylierung.

Durch Verwendung des Organodiphenylselenoxids **III** konnte eine hohe Ausbeute von 89.5% in der Hydroformylierung mit n-Octen verzeichnet werden.

Im Vergleich dazu konnte bei den Versuchen 3) und 4) die Ausbeuten weiter gesteigert werden, bei gleichbleibender guter Selektivität. Die Verwendung der erfindungsgemäßen Liganden **1a** und **1b** führt also zu einer höheren Ausbeute an Wertprodukt. Eine möglichst hohe Ausbeute ist für die Wirtschaftlichkeit von großtechnischen essentiell, da dadurch die größtmögliche Wertschöpfung aus den Rohstoffen erzielt werden kann.

Die gestellte Aufgabe konnte somit unter Verwendung der erfindungsgemäßen Liganden **1a** und **1b** erfüllt werden.

## Patentansprüche

1. Verbindung eines Organodiarylselenoxids, welches eine allgemeine Struktur (la) aufweist 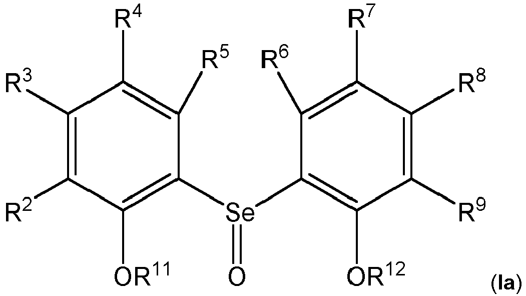
wobei R², R³, R⁴, R⁷, R⁸ und R⁹ in Struktur (la) jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl,
-CONH-(C₁-C₁₂)-Alkyl, -CO-(C₁-C₁₂)-Alkyl, -CO-(C₆-C₂₀)-Aryl, -COOH, -SO₃H, -CN,
-N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
-(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, Fluor, Chlor, Cyano, Formyl, Acyl oder Alkoxycarbonyl,
wobei R⁵ und R⁶ in Struktur (**Ia**) jeweils unabhängig voneinander ausgewählt sind aus: -H, -(C₁-C₁₂)-Alkyl, -O-(C₂-C₁₂)-Alkyl, -(C₆-C₂₀)-Aryl, -O-(C₆-C₂₀)-Aryl, -Halogen, -OC=O-(C₁-C₁₂)-Alkyl,-S-Alkyl, -S-Aryl, -COO-(C₁-C₁₂)-Alkyl,
- CONH-(C₁-C₁₂)-Alkyl, -CO-(C₁-C₁₂)-Alkyl, -CO-(C₆-C₂₀)-Aryl, -COOH, -SO₃H, -CN,
- N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
-(C₆-C₂₀)-Arylgruppen mindestens einen Substituenten aufweisen und der mindestens eine Substituent jeweils unabhängig ausgewählt ist aus -(C₃-C₁₂)-Cycloalkyl,
- (C₃-C₁₂)-Heterocycloalkyl, -(C₆-C₂₀)-Aryl, Fluor, Chlor, Cyano, Formyl, Acyl oder Alkoxycarbonyl,
wobei -R¹¹ und -R¹² jeweils in Struktur (**Ia**) unabhängig voneinander ausgewählt sind aus:
- H, -(C₁-C₁₂)-Alkyl, -(C₁-C₁₂)-Alkyl-O-(C₁-C₁₂)-Alkyl, -(C₆-C₂₀)-Aryl,
- (C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind,
wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
-(C₆-C₂₀)-Aryl, Fluor, Chlor, Cyano, Formyl, Acyl oder Alkoxycarbonyl.

2. Verbindung nach Anspruch 1, welche eine allgemeine Struktur (**Ib**) aufweist 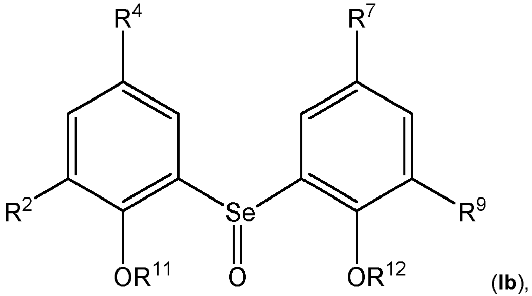 mit R², R⁴, R⁷ und R⁹ in Struktur (**Ib**) gleich -(C₁-C₁₂)-Alkyl,
wobei -R¹¹ und -R¹² jeweils in Struktur (**Ib**) unabhängig voneinander ausgewählt sind aus:
-H, -(C₁-C₁₂)-Alkyl, -(C₁-C₁₂)-Alkyl-O-(C₁-C₁₂)-Alkyl, -(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
-(C₆-C₂₀)-Aryl, Fluor, Chlor, Cyano, Formyl, Acyl oder Alkoxycarbonyl.

3. Verbindung nach Anspruch 1,
wobei in dem Organodiarylselenoxid der Strukturen (la)
R², R³, R⁴, R⁷, R⁸ und R⁹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen,
-OC=O-(C₁-C₁₂)-Alkyl, -S-Alkyl, -S-Aryl, -COO-(C₁-C₁₂)-Alkyl, -CONH-(C₁-C₁₂)-Alkyl,
-CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl-und Arylgruppen jeweils unabhängig unsubstituiert oder substituiert sind, wobei substituierte-(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
und mindestens ein Rest von R², R³, R⁴, R⁷, R⁸ und R⁹ jeweils unabhängig voneinander ausgewählt ist aus: -(C₆-C₂₀)-Aryl, -O-(C₆-C₂₀)-Aryl, -Halogen,
-OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂,
wobei die Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

4. Verbindung nach einem der Ansprüche 1 bis 3,
wobei in dem Organodiarylselenoxid
a) der Struktur (**Ia**) oder (**Ib**) R¹¹ gleich R¹² ist und ausgewählt ist aus Methoxymethyl-, Benzyl-, *tert*.-Butyl, oder
b) der Struktur (**Ia**) oder (**Ib**) R¹¹ ausgewählt ist aus Methoxymethyl-, Benzyl-, *tert*.-Butyl, und R¹² gleich - H ist, oder
c) der Struktur (**Ia**) oder (**Ib**) R¹² ausgewählt ist aus Methoxymethyl-, Benzyl-, *tert*.-Butyl und R¹¹ gleich -H ist.

5. Verbindung nach Anspruch 1,
wobei R², R³, R⁴, R⁷, R⁸, R⁹ jeweils unabhängig voneinander ausgewählt sind aus:
-H, -(C₁-C₁₂)-Alkyl, -O-(C₁-C₁₂)-Alkyl, -(C₆-C₂₀)-Aryl und -O-(C₆-C₂₀)-Aryl.

6. Verbindung nach Anspruch 1,
wobei R⁵ und R⁶ jeweils unabhängig voneinander ausgewählt sind aus:
-H, -(C₁-C₁₂)-Alkyl, -O-(C₂-C₁₂)-Alkyl, -(C₆-C₂₀)-Aryl und -O-(C₆-C₂₀)-Aryl.

7. Verbindung nach einem der Ansprüche 1 bis 6,
wobei R², R⁴, R⁷, R⁹ jeweils unabhängig voneinander ausgewählt sind aus:
-(C₁-C₁₂)-Alkyl, -O-(C₁-C₁₂)-Alkyl, -(C₆-C₂₀)-Aryl und -O-(C₆-C₂₀)-Aryl.

8. Verbindung nach einem der Ansprüche 1 bis 7,
wobei R², R⁴, R⁷, R⁹ jeweils Methyl- oder *tert*-Butyl- sind und R³, R⁵, R⁶, R⁸ sind jeweils -H.

9. Verfahren zur Herstellung eines Organodiarylselenoxids der allgemeinen Struktur (**Ia**) umfassend den Verfahrensschritt
(i) Oxidation eines Organodiarylselenids der allgemeinen Struktur (**IIa**) 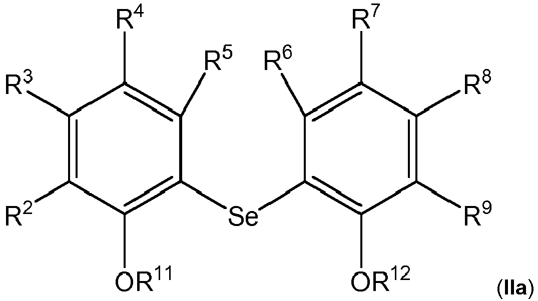
wobei R², R³, R⁴, R⁷, R⁸ und R⁹ in Struktur (la) jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl,
-CONH-(C₁-C₁₂)-Alkyl, -CO-(C₁-C₁₂)-Alkyl, -CO-(C₆-C₂₀)-Aryl, -COOH, -SO₃H, -CN,
-N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
-(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, Fluor, Chlor, Cyano, Formyl, Acyl oder Alkoxycarbonyl,
wobei R⁵ und R⁶ in Struktur (**Ia**) jeweils unabhängig voneinander ausgewählt sind aus: -H, -(C₁-C₁₂)-Alkyl, -O-(C₂-C₁₂)-Alkyl, -(C₆-C₂₀)-Aryl, -O-(C₆-C₂₀)-Aryl, -Halogen, -OC=O-(C₁-C₁₂)-Alkyl, - S-Alkyl, -S-Aryl, -COO-(C₁-C₁₂)-Alkyl,
-CONH-(C₁-C₁₂)-Alkyl, -CO-(C₁-C₁₂)-Alkyl, -CO-(C₆-C₂₀)-Aryl, -COOH, -SO₃H, -CN,
-N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
-(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, Fluor, Chlor, Cyano, Formyl, Acyl oder Alkoxycarbonyl,
wobei -R¹¹ und -R¹² jeweils in Struktur (**Ia**) unabhängig voneinander ausgewählt sind aus:
-H, -(C₁-C₁₂)-Alkyl, -(C₁-C₁₂)-Alkyl-O-(C₁-C₁₂)-Alkyl, -(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind,
wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
-(C₆-C₂₀)-Aryl, Fluor, Chlor, Cyano, Formyl, Acyl oder Alkoxycarbonyl,
(ii) mindestens eine Verbindung eines Organodiarylselenoxids der allgemeinen Struktur (**Ia**) erhalten wird, 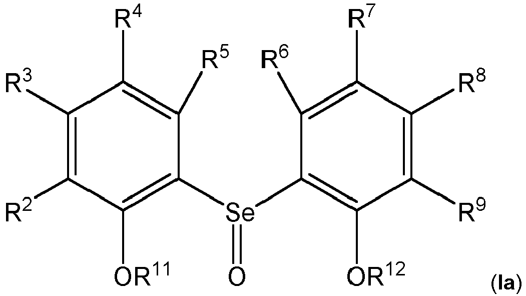
wobei R², R³, R⁴, R⁷, R⁸ und R⁹ in Struktur (**Ia**) jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, - OC=O-(C₁-C₁₂)-Alkyl, -S-Alkyl, -S-Aryl, -COO-(C₁-C₁₂)-Alkyl,
-CONH-(C₁-C₁₂)-Alkyl, -CO-(C₁-C₁₂)-Alkyl, -CO-(C₆-C₂₀)-Aryl, -COOH, -SO₃H, -CN,
-N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
-(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, Fluor, Chlor, Cyano, Formyl, Acyl oder Alkoxycarbonyl,
wobei R⁵ und R⁶ in Struktur (**Ia**) jeweils unabhängig voneinander ausgewählt sind aus: -H, -(C₁-C₁₂)-Alkyl, -O-(C₂-C₁₂)-Alkyl, -(C₆-C₂₀)-Aryl, -O-(C₆-C₂₀)-Aryl, -Halogen, -OC=O-(C₁-C₁₂)-Alkyl, - S-Alkyl, -S-Aryl, -COO-(C₁-C₁₂)-Alkyl,
-CONH-(C₁-C₁₂)-Alkyl, -CO-(C₁-C₁₂)-Alkyl, -CO-(C₆-C₂₀)-Aryl, -COOH, -SO₃H, -CN,
-N[(C₁-C₁₂)-Alkyl]₂, wobei die Alkyl- und Arylgruppen jeweils unabhängig unsubstituiert oder substituiert sind, wobei substituierte -(C₁-C₁₂)-Alkylgruppen und substituierte
-(C₆-C₂₀)-Arylgruppen mindestens einen Substituenten aufweisen und der mindestens eine Substituent jeweils unabhängig ausgewählt ist aus -(C₃-C₁₂)-Cycloalkyl,
-(C₃-C₁₂)-Heterocycloalkyl, -(C₆-C₂₀)-Aryl, Fluor, Chlor, Cyano, Formyl, Acyl oder Alkoxycarbonyl,
wobei -R¹¹ und -R¹² jeweils in Struktur (**Ia**) unabhängig voneinander ausgewählt sind aus:
-H, -(C₁-C₁₂)-Alkyl, -(C₁-C₁₂)-Alkyl-O-(C₁-C₁₂)-Alkyl, -(C₆-C₂₀)-Aryl,
-(C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind,
wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl,
-(C₆-C₂₀)-Aryl, Fluor, Chlor, Cyano, Formyl, Acyl oder Alkoxycarbonyl.

10. Verfahren nach Anspruch 9,
wobei (i) das Oxidationsmittel zur Oxidation des Organodiarylselenoxid der allgemeinen Struktur (**II**) umfasst N-Chlorsuccinimid (CNS), Bromsuccinimid (BNS), Wasserstoffperoxid, *tert*-Butylhypochlorit (tBuOCl), Natriumhypochlorit (NaOCl) und/oder meta-Chlorbenzoesäure (mCPBA).

11. Komplex umfassend
- mindestens ein Organodiarylselenoxid einer der Ansprüche 1 bis 8 und
- mindestens ein Metallatom ausgewählt aus Rh, Ru, Co, Ir.

12. Verwendung mindestens einer Verbindung nach einem der Ansprüche 1 bis 8,
a) als Ligand in einem Komplex umfassend mindestens ein Metallatom oder
b) zur Katalyse einer Hydroformylierungsreaktion.

13. Verfahren umfassend die Verfahrensschritte
(i) Vorlegen mindestens eines Olefins,
(ii) Zugabe eines Komplexes nach Anspruch 11,
oder ein Organodiarylselenoxid nach einem der Ansprüche 1 bis 8 und einer Substanz, welche ein Metallatom ausgewählt aus Rh, Ru, Co, Ir aufweist,
(iii) Zuführen von H₂ und CO,
(iv) Erwärmen des Reaktionsgemisches,
wobei das Olefin zu einem Aldehyd umgesetzt wird.

## Geänderte Patentansprüche

### Geänderte Patentansprüche gemäss Regel 137(2) EPÜ.

1. Verfahren umfassend die Verfahrensschritte
(i) Vorlegen mindestens eines Olefins,
(ii) Zugabe eines Komplexes umfassend
- mindestens ein Organodiarylselenoxid der allgemeinen Struktur (**Ia***): 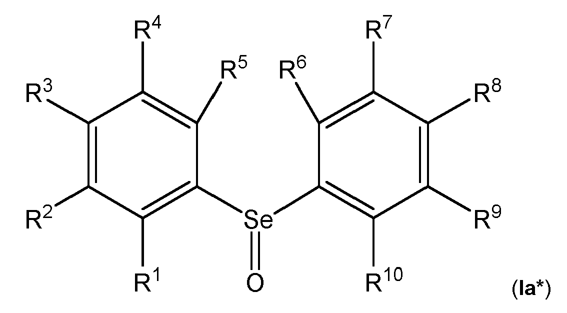
wobei R², R³, R⁴, R⁵, R⁶, R⁷, R⁸ und R⁹ jeweils unabhängig voneinander ausgewählt sind aus: - H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl- und Arylgruppen jeweils unsubstituiert sind, und
wobei -R¹ gleich -OR¹¹ und -R¹⁰ gleich -OR¹² sind, wobei R¹¹ und R¹² jeweils unabhängig voneinander ausgewählt sind aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
und
- mindestens ein Metallatom ausgewählt aus Rh, Ru, Co, Ir; oder ein Organodiarylselenoxid der allgemeinen Struktur: 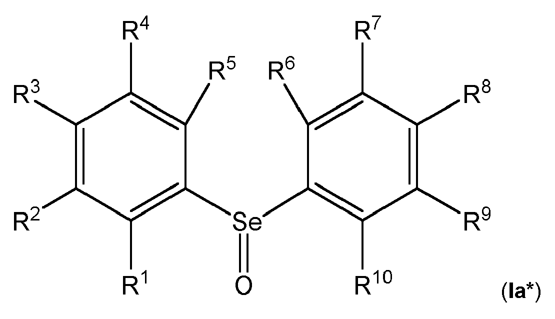
wobei R², R³, R⁴, R⁵, R⁶, R⁷, R⁸ und R⁹ jeweils unabhängig voneinander ausgewählt sind aus: - H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkyl-und Arylgruppen jeweils unsubstituiert sind, und
wobei -R¹ gleich -OR¹¹ und -R¹⁰ gleich -OR¹² sind, wobei R¹¹ und R¹² jeweils unabhängig voneinander ausgewählt sind aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₆-C₂₀)-Aryl, -C=O-(C₁-C₁₂)-Alkyl, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
und einer Substanz, welche ein Metallatom ausgewählt aus Rh, Ru, Co, Ir aufweist,
(iii) Zuführen von H₂ und CO,
(iv) Erwärmen des Reaktionsgemisches,
wobei das Olefin zu einem Aldehyd umgesetzt wird.
